# EUROPÄISCHE PATENTSCHRIFT

(11) **EP 2 360 138 B1**
(45) Veröffentlichungstag und Bekanntmachung des Hinweises auf die Patenterteilung: **31.10.2018**
(21) Anmeldenummer: 10173421.8
(22) Anmeldetag: 31.08.2006
(51) Int. Cl.: C07C 51/41, C07C 57/04, B01J 14/00, C08F 20/06, C08F 220/06

(54) **VORRICHTUNG ZUR DURCHFÜHRUNG EINES KONTINUIERLICHEN NEUTRALISATIONSVERFAHRENS**
Apparatus for cyrrying out a continuous neutralization process
Appareil pour executer un procédé continu de neutralisation

(30) Priorität: 07.09.2005 DE 102005042604
(43) Veröffentlichungstag der Anmeldung: 24.08.2011
(62) Teilanmeldung aus: 06793091.7
(73) Patentinhaber: BASF SE, 67056 Ludwigshafen am Rhein (DE)
(72) Erfinder: Weismantel, Matthias, 63637 Jossgrund (DE); De Marco, Michael, 69469 Weinheim (DE); van Esbroeck, Dominicus, 210019 Nanjing (CN); Possemiers, Karl, J., 2970 Schilde (BE); De Kaey, Ronny, 2531 Vremde (BE)
(74) Vertreter: BASF IP Association

(56) Entgegenhaltungen:
- EP-A1- 0 028 203
- WO-A1-2007/028746
- FR-A- 1 367 524
- US-A- 3 178 481

## Beschreibung

Die vorliegende Erfindung betrifft eine Vorrichtung zur kontinuierlichen Neutralisation.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure bevorzugt werden. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Herstellung der wasserabsorbierenden Polymere wird beispielsweise in der Monographie "Modern Superabsorbent Polymer Technology", F.L. Buchholz und A.T. Graham, Wiley-VCH, 1998, oder in Ullmann's Encyclopedia of Industrial Chemistry, 6. Auflage, Band 35, Seiten 73 bis 103, beschrieben. Das bevorzugte Herstellungsverfahren ist die Lösungs- oder Gelpolymerisation. Bei dieser Technologie wird zunächst eine Monomermischung hergestellt, die diskontinuierlich neutralisiert und dann in einen Polymerisationsreaktor überführt wird, oder bereits im Polymerisationsreaktor vorgelegt wird. Im sich anschließenden diskontinuierlichen oder kontinuierlichen Verfahren erfolgt die Reaktion zum Polymergel, das im Falle einer gerührten Polymerisation bereits zerkleinert wird. Das Polymergel wird anschließend getrocknet, gemahlen und gesiebt und dann zur weiteren Oberflächenbehandlung transferiert.

Ein kontinuierliches Polymerisationsverfahren liegt beispielsweise der WO 01/38402 zugrunde, wobei die wässrige Monomerlösung kontinuierlich einem Mischkneter mit mindestens zwei achsparallel rotierenden Wellen zugeführt wird.

Kontinuierliche Gelpolymerisationen sind weiterhin bekannt aus WO 03/004237, WO 03/022896 und WO 01/016197.

Sowohl bei der kontinuierlichen als auch bei der diskontinuierlichen Polymerisation wird die Acrylsäure im Falle der Vorneutralisation diskontinuierlich neutralisiert. Typischerweise werden im Polymerisationsreaktor im Falle der Lösungspolymerisation die Edukte Acrylsäure, Wasser, optionale Comonomere und Natronlauge diskontinuierlich dosiert und vermischt. In diesem Schritt wird weitestgehend der restliche Polymerisationsverlauf und auch die zu erwartenden Polymereigenschaften festgelegt. Der Vernetzungsgrad des Basispolymers sowie der Neutralisationsgrad werden typischerweise in diesem Schritt bestimmt. Der Neutralisationsgrad der Monomeren liegt zwischen 0 und 80 mol-%. Im Falle der sauren Polymerisation wird das erhaltene Polymergel üblicherweise zu 50 bis 80 mol-%, vorzugsweise zu 60 bis 75 mol-% nachneutralisiert, indem Natriumhydroxid- oder Natriumcarbonatlösung zum sauren Polymergel zugegeben und eingearbeitet wird.

Neutralisationsverfahren werden beispielsweise in EP-A 0 372 706, EP-A 0 574 260, WO 03/051415 und EP-A 1 470 905 beschrieben.

EP-A 0 372 706 beschreibt ein dreistufiges Neutralisationsverfahren, bei dem in einer ersten Stufe Acrylsäure und Natronlauge gleichzeitig dosiert werden, so dass ein Neutralisationsgrad von 75 bis 100 mol-% eingehalten wird, in einer zweiten Stufe der Neutralisationsgrad auf 100,1 bis 110 mol-% angehoben wird um in der eingesetzten Acrylsäure als Verunreinigung enthaltene Diacrylsäure zu hydrolysieren, und in einer dritten Stufe durch Zusatz von weiterer Acrylsäure ein Neutralisationsgrad von 20 bis 100 mol-% eingestellt wird.

EP-A 0 574 260 offenbart auf Seite 7, Zeilen 38 bis 41, dass bei der Neutralisation vorteilhaft Natronlauge vorgelegt und anschließend Acrylsäure unter Kühlung zugesetzt wird.

WO 03/051415 lehrt ein Verfahren zur Herstellung wasserabsorbierender Polymere, bei dem die Monomerlösung eine Mindesttemperatur von 40°C aufweist.

EP-A 1 470 905 beschreibt in den Beispielen die kontinuierliche Neutralisation von Acrylsäure unmittelbar vor dem Polymeriationsreaktor. Aufgrund der Neutralisationswärme steigt die Temperatur auf 95°C.

Es ist bekannt, dass sich die Reaktivität der Acrylsäure sehr stark von der ihrer Salze unterscheidet, weshalb auch der Polymerisationsverlauf stark abhängig ist vom pH-Wert, bei dem er stattfindet. In einer anschaulichen Darstellung in der Monographie "Modern Superabsorbent Polymer Technology", F.L. Buchholz und A.T. Graham, Wiley-VCH, 1998, oder in Ullmann's Encyclopedia of Industrial Chemistry, 6. Auflage, Band 35, Seite 35, ist die Polymerisationsgeschwindigkeit als Funktion des pH-Wertes aufgetragen. Demnach durchläuft die Polymerisationsgeschwindigkeit ein Minimum bei einem pH-Wert von 6 bis 7. Dies entspricht allerdings dem pH-Wert, der in aller Regel bei den Verkaufsprodukten gewünscht ist. Erklärt wird dieses Verhalten dadurch, dass es zwischen dem geladenen Monomeren in Salzform und der wachsenden Radikalkette zu elektrostatischen Abstoßungsreaktionen kommt, die im Falle der weitestgehend undissoziierten Acrylsäure nicht vorliegen, was zur Verlangsamung des Reaktionsverlaufs führt.

Es ist auch bekannt, dass unneutralisierte Acrylsäure leichter polymerisiert werden kann als vorneutralisierte Systeme. Diese Differenz wird jedoch bei steigender Monomerkonzentration verringert, vor allem deswegen, weil eine höhere Monomerkonzentration die Dissoziation der Acrylsäuresalze unterdrückt.

Um all diesen reaktionstechnischen Details Rechnung zu tragen, werden bei der Reaktionsführung üblicherweise Kompromisse eingegangen.

Generell wird der Neutralisationsgrad der Acrylsäure bereits vor dem Eintritt in die kontinuierliche Polymerisation eingestellt. Die Neutralisation erfolgt diskontinuierlich. Die diskontinuierliche Neutralisation hat den Vorteil, dass die Dosierung von Acrylsäure und/oder Natronlauge temperaturgeregelt erfolgen kann. Damit werden Überhitzungen und unerwünschte Polymerisationen in dem Ansatzbehälter vermieden. Der Neutralisationsgrad wird entsprechend den Polymerisationsbedingungen und dem erwünschten Absorptionsprofil gewählt und wahlweise in einer nachgeschalteten Neutralisation, die zumeist am Polymergel erfolgt, korrigiert.

Nachteil der Neutralisation als vorgeschalteter diskontinuierlicher Verfahrensschritt ist vor allem der logistische Aufwand. Die Bereitstellung von Vorratstanks, Kesseln oder Behältnissen, welche die teilneutralisierte Acrylsäure bevorraten, sowie der komplette apparative Aufwand wirken sich nachteilig auf die Wirtschaftlichkeit des Prozesses aus.

Erwünschenswert wäre es daher, die Neutralisation kontinuierlich durchzuführen. Die kontinuierliche Neutralisation könnte dann vorteilhaft mit einer kontinuierlichen Polymerisation kombiniert werden, wobei der logistische Aufwand und die Bereitstellung von Vorratsbehältern minimiert wird. Die kontinuierliche Neutralisation erweist sich jedoch aufgrund der auftretenden Wärmeentwicklung als problematisch, da es dadurch zur unerwünschten vorzeitigen Polymerisation kommt. Der weitere Verlauf der kontinuierlichen Polymerisation wird durch bereits entstandenes Polymergel in allen Fällen negativ beeinträchtigt, insbesondere durch Verstopfungen in Leitungen, die nur für den Flüssigkeitstransport ausgelegt wurden.

Aufgabe der vorliegenden Erfindung war die Bereitstellung einer Vorrichtung zur kontinuierlichen Neutralisation, wobei das Auftreten unerwünscht hoher Temperaturspitzen vermieden werden kann.

Gelöst wurde die Aufgabe durch eine Vorrichtung zur kontinuierlichen Neutralisation, umfassend
i) eine Ringleitung R,
ii) mindestens eine erste Zuleitung Z₁ in die Ringleitung R,
iii) mindestens eine zweite Zuleitung Z₂ in die Ringleitung R,
iv) mindestens einen Wärmeaustauscher W in der Ringleitung R, wobei sich der Wärmeaustauscher W in Flussrichtung hinter den Zuleitungen Z₁ und Z₂ befindet,
v) mindestens eine Ableitung A aus der Ringleitung R, wobei sich die Ableitung A in Flussrichtung hinter dem Wärmeaustauscher W befindet,
vi) eine Pumpe P und
vii) einen Behälter B zwischen dem Wärmeaustauscher W und der Ableitung A,
wobei mindestens eine erste Zuleitung Z₁ bedeutet, dass das Edukt 1, beispielsweise Natronlauge, über eine oder mehrere Zuleitungen Z₁ zugeführt wird, und mindestens eine zweite Zuleitung Z₂ bedeutet, dass das Edukt 2, beispielsweise Acrylsäure, über eine oder mehrere Zuleitungen Z₂ zugeführt wird.

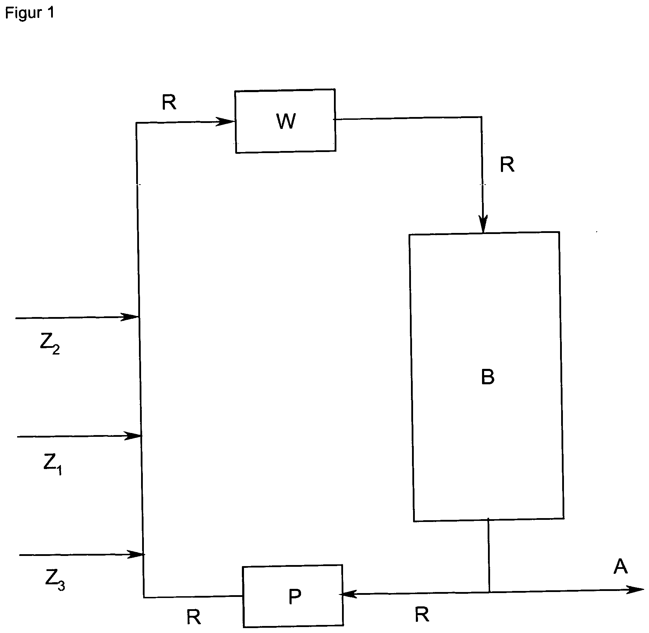

Eine beispielhafte Neutralisation zeigt Figur 1, wobei die Bezugszeichen folgende Bedeutungen haben:
- Z₁ bis Z₃: Zuführungen für Edukte 1 bis 3
- A: Ableitung
- B: Behälter
- P: Pumpe
- R: Ringleitung
- W: Wärmeaustauscher

Mittels einer Pumpe P wird neutralisierte Lösung teilweise über die Ringleitung R rückgeführt. Der Rest der neutralisierten Lösung wird über die Ableitung A der weiteren Verwendung zugeführt. Der Behälter B dient als Puffer. Über die Zuleitung Z₁ wird vorzugsweise 50gew.-%ige Natronlauge, über die Zuleitung Z₂ wird vorzugsweise Acrylsäure und über die Zuleitung Z₃ wird vorzugsweise Wasser dosiert.

Der Ringleitungsquerschnitt Q beträgt von vorzugsweise von 20 bis 2000 cm², besonders bevorzugt von 80 bis 700 cm², ganz besonders bevorzugt von 200 bis 500 cm². Die Ringleitung R hat vorzugsweise einen kreisrunden Querschnitt.

Die Summe der Zuleitungen Z₁ hat einen Querschnitt vorzugsweise von 1,5 bis 100 cm², besonders bevorzugt von 6 bis 35 cm², ganz besonders bevorzugt von 15 bis 25 cm². Die Zuleitungen Z₁ haben vorzugsweise einen kreisrunden Querschnitt.

Die Summe der Zuleitungen Z₂ hat einen Querschnitt vorzugsweise von 1,5 bis 100 cm², besonders bevorzugt von 6 bis 35 cm², ganz besonders bevorzugt von 15 bis 25 cm². Die Zuleitungen Z₂ haben vorzugsweise einen kreisrunden Querschnitt.

Die Pumpe P hat eine Förderleistung von vorzugsweise 1 bis 1000 t/h, besonders bevorzugt von 10 bis 700 t/h, ganz besonders bevorzugt von 100 bis 500 t/h.

Der Behälter B hat ein Volumen von vorzugsweise 1 bis 100 m³, besonders bevorzugt von 10 bis 100 m³, ganz besonders bevorzugt von 20 bis 50 m³.

Die Zuleitungen Z₁ und Z₂ sind hintereinander angeordnet, wobei die Zuleitungen Z₁ in Flussrichtung vor den Zuleitungen Z₂ liegen.

Der Abstand zwischen den Zuleitungen Z₁ und Z₂ beträgt vorzugsweise 10 bis 500%, besonders bevorzugt 50 bis 300%, ganz besonders bevorzugt 80 bis 200%, der Quadratwurzel des Ringleitungsquerschnitts Q.

Es sind vorzugsweise mindestens zwei Zuleitungen Z₁ und/oder Z₂ vorhanden, besonders bevorzugt zwei, drei, vier, fünf oder sechs Zuleitungen Z₁ bzw. Z₂, wobei die Zuleitungen Z₁ bzw. Z₂ vorzugsweise so angeordnet sind, dass sie eine gemeinsame Achse aufweisen (für zwei Zuleitungen Z₁ bzw. Z₂) oder einen symmetrischen Stern bilden (für mindestens drei Zuleitungen Z₁ bzw. Z₂) und die Achse bzw. Stern senkrecht zur Flussrichtung der neutralisierten Lösung befindet (Mehrfachzugabestellen).

Besonders vorteilhaft werden zwei, drei oder vier Mehrfachzugabestellen hintereinander angeordnet.

Beispielsweise können mindestens acht Zuleitungen Z₁ vorhanden sein, wobei jeweils vier Zuleitungen Z₁ kreuzförmig in die Ringleitung R münden, die mindestens 2 Vierergruppen an Zuleitungen Z₁ hintereinander und gegeneinander versetzt angeordnet sind.

Weiterhin kann mindestens eine dritte Zuleitung Z₃ in die Ringleitung R münden, wobei mindestens eine dritte Zuleitung Z₃ bedeutet, dass das Edukt 3, beispielsweise Wasser, über eine oder mehrere Zuleitungen Z₃ zugeführt wird, und die Zuleitung Z₃ in Flussrichtung vor der Zuleitung Z₁ liegt und/oder die Zuleitung Z₁ umhüllt.

Der Abstand zwischen den Zuleitungen Z₃ und Z₁ beträgt vorzugsweise 10 bis 500%, besonders bevorzugt 50 bis 300%, ganz besonders bevorzugt 80 bis 200%, der Quadratwurzel des Ringleitungsquerschnitts Q.

Vorzugsweise ist die Ringleitung R an mindestens einer Zuleitung Z₁ bis Z₃ als Venturi-Rohr ausgebildet.

Die Zuleitungen münden besonders bevorzugt alle in ein gemeinsames Venturi-Rohr.

Die Vorrichtung ist vorzugsweise totraumfrei, bevorzugte Werkstoffe sind Edelstähle, beispielsweise die Werkstoffe Nr. 1.4541 und Nr. 1.4571 gemäß DIN 17007.

Die Oberflächen sollten eine möglichst geringe Rauhigkeit aufweisen.

Toträume sind Abschnitte der Vorrichtung in denen die durchschnittliche Verweilzeit beim bestimmungsgemäßen Betrieb erhöht ist.

### Beispiele

Die Peaktemperatur der folgenden Beispiele wurde mit Fluent 6.0 berechnet. Die Peaktemperatur ist die höchste im System auftretende Temperatur. Das Programm kann beispielsweise über Fluent Deutschland GmbH, Birkenweg 14a, D-64295 Darmstadt, bezogen werden. Weitere Bezugsquellen sind unter www.fluent.com zu finden.

### Beispiele 1 bis 6

Für die Beispiele 1 bis 6 wurde eine Vorrichtung, wie in Figur 1 beschrieben, angenommen. Für die Berechnung wurde der Durchmesser der Ringleitung R zu 20 cm, der Durchmesser der Zuführungen Z₁ bis Z₃ jeweils zu 5 cm, der Massenstrom in der Ringleitung R vor der Zuführung Z₃ zu 349 t/h, die Temperatur des Massenstromes in der Ringleitung R vor der Zuführung Z₃ zu 26°C, der Abstand zwischen Zuführung Z₃ und Zuführung Z₁ zu 20 cm, der Abstand zwischen Zuführung Z₁ und Zuführung Z₂ zu 20 cm, der Massenstrom Acrylsäure zu 11,5 t/h, der Massenstrom 50gew.-%ige Natronlauge zu 8,4 t/h und der Massenstrom Wasser zu 15 t/h festgelegt.

Die Beispiele 1 bis 3 belegen den Einfluss der Dosierreihenfolge.

In den Beispielen 4 bis 6 werden Natronlauge und Wasser vorgemischt. In den Beispielen 5 und 6 wurde die Berechnung zusätzlich um eine Kühlung des Natronlauge/Wasser-Gemisches vor dem Eintritt in die Ringleitung R auf die angegebene Temperatur ergänzt.

Die Beispiele belegen, wie über die Dosierreihenfolge die Peaktemperatur beeinflusst werden kann.

| Beispiel | Zuführung | | | Temperatur | | | Peaktemperatur |
|---|---|---|---|---|---|---|---|
| | Z₃ | Z₁ | Z₂ | NaOH | H₂O | AS | |
| 1 | H₂O | AS | NaOH | 23°C | 23°C | 23°C | 64°C |
| 2 | NaOH | H₂O | AS | 23°C | 23°C | 23°C | 47°C |
| 3 | AS | H₂O | NaOH | 23°C | 23°C | 23°C | 64°C |
| 4 | - | NaOH/H₂O | AS | 23°C | 23°C | 23°C | 43°C |
| 5 | - | NaOH/H₂O | AS | 23°C*) | 23°C*) | 15°C | 38°C |
| 6 | - | NaOH/H₂O | AS | 15°C*) | 15°C*) | 15°C | 35°C |

| | | | | | | | |
|---|---|---|---|---|---|---|---|
| NaOH: 50gew.-%ige Natronlauge H₂O: Wasser AS: Acrylsäure *) Temperatur der NaOH/H₂O-Mischung | | | | | | | |

### Beispiele 7 bis 9

Die Beispiele 7 bis 9 wurden analog zu den Beispielen 1 bis 6 berechnet.

Für die Berechnung der Beispiele 7 bis 9 wurde angenommen, dass eine NaOH/H₂O-Mischung und Acrylsäure in ein gemeinsames Venturi-Rohr dosiert werden. Die Länge des Venturi-Rohres wurde zu 93,2 cm festgelegt, wobei sich das Venturi-Rohr über eine Strecke von 8,4 cm auf einen Durchmesser von 10 cm verjüngt, über eine Strecke von 27,6 cm den Durchmesser von 10 cm beibehält und sich über eine Strecke von 57 cm wieder auf einen Durchmesser von 20 cm weitet. Der Abstand der Zuführung Z₁ von der Stelle, wo sich das Venturi-Rohr auf 10 cm verjüngt hat, wurde zu 5 cm, der Abstand der Zuführung Z₂ von der Zuführung Z₁ wurde zu 8 cm und der Durchmesser der jeweils zwei Zuführungen Z₁ und Z₂ wurde zu 3,5 cm angenommen. Die jeweils beiden Zuführungen Z₁ bzw. Z₂ sind gegenüber angeordnet, wobei die Verbindungsachse der beiden Zuführungen Z₁ gegenüber der Verbindungsachse der beiden Zuführungen Z₂ um 90° gedreht ist.

In den Beispielen 7 bis 9 wurde zusätzlich der Einfluss des Massenstromes in der Ringleitung R untersucht. In den Beispielen 8 und 9 wurde der Massenstrom in der Ringleitung R daher rechnerisch um 50% bzw. 90% reduziert.

| Beispiel | Mischungsverhältnis | Zuführung | | Temperatur | | Peaktemperatur |
|---|---|---|---|---|---|---|
| | | Z₁ | Z₂ | NaOH/H₂O | AS | |
| 7 | 1 : 10 | NaOH/H₂O | AS | 15°C | 15°C | 27°C |
| 8 | 1 : 5 | NaOH/H₂O | AS | 15°C | 15°C | 46°C |
| 9 | 1 : 1 | NaOH/H₂O | AS | 15°C | 15°C | 69°C |

Die Berechnungen belegen auch die Vorteile des Venturi-Rohres gegenüber der einfachen Dosierung (Vergleich der Beispiele 6 und 7).

## Patentansprüche

1. Vorrichtung zur kontinuierlichen Neutralisation, umfassend
i) eine Ringleitung R,
ii) mindestens eine erste Zuleitung Z₁ in die Ringleitung R,
iii) mindestens eine zweite Zuleitung Z₂ in die Ringleitung R,
iv) mindestens einen Wärmeaustauscher W in der Ringleitung R, wobei sich der Wärmeaustauscher W in Flussrichtung hinter den Zuleitungen Z₁ und Z₂ befindet,
v) mindestens eine Ableitung A aus der Ringleitung R, wobei sich die Ableitung A in Flussrichtung hinter dem Wärmeaustauscher W befindet,
vi) eine Pumpe P und
vii) einen Behälter B zwischen dem Wärmeaustauscher W und der Ableitung A,
wobei sich die Zuleitung Z₂ in Flussrichtung hinter der Zuleitung Z₁ befindet.

2. Vorrichtung gemäß Anspruch 1, **dadurch gekennzeichnet, dass** der Abstand zwischen den Zuleitungen Z₁ und Z₂ von 10 bis 500% der Quadratwurzel des Ringleitungsquerschnitts beträgt.

3. Vorrichtung gemäß Anspruch 1 oder 2, **dadurch gekennzeichnet, dass** mindestens zwei Zuleitungen Z₁ und/oder Z₂ vorhanden sind.

4. Vorrichtung gemäß einem der Ansprüche 1 bis 3, **dadurch gekennzeichnet, dass** mindestens acht Zuleitungen Z₁ vorhanden sind, wobei jeweils vier Zuleitungen Z₁ kreuzförmig in die Ringleitung R münden, die mindestens 2 Vierergruppen an Zuleitungen Z₁ hintereinander und gegeneinander versetzt angeordnet sind.

5. Vorrichtung gemäß einem der Ansprüche 1 oder 4, **dadurch gekennzeichnet, dass** mindestens eine dritte Zuleitung Z₃ in die Ringleitung R mündet, wobei die Zuleitung Z₃ in Flussrichtung vor der Zuleitung Z₁ liegt und/oder die Zuleitung Z₁ umhüllt.

6. Vorrichtung gemäß einem der Ansprüche 1 oder 5, **dadurch gekennzeichnet, dass** die Ringleitung R an der Einmündung mindestens einer Zuleitung als Venturi-Rohr ausgebildet ist.

7. Vorrichtung gemäß Anspruch 6, **dadurch gekennzeichnet, dass** die Zuleitungen in ein gemeinsames Venturi-Rohr münden.

## Claims

1. An apparatus for continuous neutralization, comprising
i) a ring line R,
ii) at least one first inlet Z₁ into the ring line R,
iii) at least one second inlet Z₂ into the ring line R,
iv) at least one heat exchanger W in the ring line R, the heat exchanger fW being disposed beyond the inlets Z₁ and Z₂ in flow direction,
v) at least one outlet A from the ring line R, the outlet A being disposed beyond the heat exchanger W in flow direction,
vi) a pump P and
vii) a vessel B between the heat exchanger W and the outlet A,
wherein inlet Z₂ is disposed beyond inlet Z₁ in flow direction.

2. The apparatus according to claim 1, wherein the distance between the inlets Z₁ and Z₂ is from 10 to 500% of the square root of the ring line cross section.

3. The apparatus according to claim 1 or 2, wherein at least two inlets Z₁ and/or Z₂ are present.

4. The apparatus according to any of claims 1 to 3, wherein at least eight inlets Z₁ are present, in which case four inlets Z₁ in each case open in a cross shape into the ring line R, the at least 2 groups of four inlets Z₁ being arranged in succession and offset relative to one another.

5. The apparatus according to either of claims 1 and 4, wherein at least one third inlet Z₃ opens into the ring line R, in which case inlet Z₃ is before inlet Z₁ in flow direction and/or encloses inlet Z₁.

6. The apparatus according to either of claims 1 and 5, wherein the ring line R is designed as a Venturi tube at the opening of at least one inlet.

7. The apparatus according to claim 6, wherein the inlets open into a common Venturi tube.

## Revendications

1. Dispositif de neutralisation continue, comprenant
i) un conduit annulaire R,
ii) au moins un premier conduit d'alimentation Z₁ dans le conduit annulaire R,
iii) au moins un deuxième conduit d'alimentation Z₂ dans le conduit annulaire R,
iv) au moins un échangeur thermique W dans le conduit annulaire R, dans la direction du flux, l'échangeur thermique W se trouvant derrière les conduits d'alimentation Z₁ et Z₂,
v) au moins un conduit d'évacuation A hors du conduit annulaire R, dans la direction du flux, le conduit d'évacuation A se trouvant derrière l'échangeur thermique W,
vi) une pompe P et
vii) un réservoir B entre l'échangeur thermique W le conduit d'évacuation A,
dans la direction du flux, le conduit d'alimentation Z₂ se trouvant derrière le conduit d'alimentation Z₁.

2. Dispositif selon la revendication 1, **caractérisé en ce que** l'écart entre les conduits d'alimentation Z₁ et Z₂ est de 10 à 500 % de la racine carrée de la section transversale du conduit annulaire.

3. Dispositif selon la revendication 1 ou 2, **caractérisé en ce qu'**au moins deux conduits d'alimentation Z₁ et/ou Z₂ sont présents.

4. Dispositif selon l'une quelconque des revendications 1 à 3, **caractérisé en ce qu'**au moins huit conduits d'alimentation Z₁ sont présents, chaque fois quatre conduits d'alimentation Z₁ débouchant sous forme cruciforme dans le conduit annulaire R, les au moins 2 groupes de quatre conduits d'alimentation Z₁ étant placés l'un derrière l'autre et avec un décalage mutuel.

5. Dispositif selon l'une quelconque des revendications 1 ou 4, **caractérisé en ce qu'**au moins un troisième conduit d'alimentation Z₃ débouche dans le conduit annulaire R, dans la direction du flux, le conduit d'alimentation Z₃ se situant à l'avant du conduit d'alimentation Z₁ et/ou enveloppant le conduit d'alimentation Z₁.

6. Dispositif selon l'une quelconque des revendications 1 ou 5, **caractérisé en ce que** sur l'embouchure d'au moins un conduit d'alimentation, le conduit annulaire R est conçu sous la forme d'un tube Venturi.

7. Dispositif selon la revendication 6, caractérisé en ce les conduits d'alimentation débouchent dans un tube Venturi commun.
